# EUROPEAN PATENT SPECIFICATION

(11) **EP 3 996 605 B1**
(45) Date of publication and mention of the grant of the patent: **08.01.2025**
(21) Application number: 19745079.4
(22) Date of filing: 22.07.2019
(51) Int. Cl.: A61B 17/12

(54) **AN EMBOLIZATION DEVICE FOR PROMOTING CLOT FORMATION IN A BODILY LUMEN AND A METHOD OF ASSEMBLING SAID EMBOLIZATION DEVICE**
EMBOLISATIONSVORRICHTUNG ZUR FÖRDERUNG DER GERINNSELBILDUNG IN EINEM KÖRPERLUMEN UND VERFAHREN ZUM ZUSAMMENBAU DIESER EMBOLISATIONSVORRICHTUNG
DISPOSITIF D'EMBOLISATION POUR FAVORISER LA FORMATION DE CAILLOT DANS UNE LUMIÈRE CORPORELLE ET PROCÉDÉ D'ASSEMBLAGE DUDIT DISPOSITIF D'EMBOLISATION

(43) Date of publication of application: 18.05.2022
(73) Proprietor: Clearstream Technologies Limited, Enniscorthy, County Wexford (IE)
(72) Inventor: BRADY, Conor, Enniscorthy, County Wexford (IE)
(74) Representative: Hoffmann Eitle
(86) International application number: PCT/EP2019/069663
(87) International publication number: WO 2021/013330

(56) References cited:
- EP-A1- 2 893 902
- WO-A2-2016/041961
- US-A1- 2014 276 388
- US-A1- 2016 166 257
- US-B1- 8 690 907

## Description

### Technical Field

The present disclosure relates to an embolization device for promoting clot formation in a bodily lumen and having a contracted delivery configuration and an expanded deployed configuration, the embolization device having a stem and a plurality of flexible bristles extending radially outwardly from the stem, and a method of assembling such an embolization device.

### Background

An embolization device is a permanent or semi-permanent implantable device that may be received within a bodily lumen so as to promote clot formation therein.

Embolization devices may be deployed in the vasculature at a particular location by a medical practitioner so as to promote blood clot formation and ultimately occlude the blood vessel. However, typical embolization devices may be prone to migration within the vasculature which may cause serious adverse effects.

To reduce migration, some known embolization devices, as disclosed for example in WO 2016/041961 A2, comprise a number of bristles or fibres extending radially outwardly from a central stem. The bristles are configured to contact the bodily lumen and anchor the embolization device in the lumen due to friction between the bristles and the wall of the lumen.

These embolization devices consist of a number of segments which are connected by a solid hypotube which is crimped to the distal end of a first segment and the proximal end of a second segment. This hypotube is made from a similar material to the stem wire but diameter and wall thickness render it much stiffer than the deformed wire in the stem.

When the embolization device is implanted into a bodily lumen of a patient, the embolization device may have to undergo significant bending to navigate the tortuous blood vessels of the patient's body. When a bending moment is induced in the in the stem of the embolization device, stress concentrations occur just outside the ends of the hypotube. In some cases, this may cause the stem wire to fracture at these locations. Once fracture occurs, the segments are no longer constrained and may be able to move relative to each other, increasing the risk of component migration, vessel perforation and, if the segment turns sideways, recanalization.

In view of the above, there is a need for an improved embolization device which can distribute the stress concentrations during bending more evenly and is therefore less prone to fracture. There is also a need for an improved method of assembling such an embolization device.

US 2016/166257 A1 discloses a bristle device for delivery into a body lumen comprises a longitudinally extending stem and a plurality of bristles extending generally outwardly from the stem for anchoring the device in a body lumen. There may be at least two bristle segments and in some cases there are flexible sections between the segments. The flexible sections articulate to enable the device to pass through a catheter placed in a tortuous anatomy or to be deployed in a curved vessel, or across a bifurcation. In some cases at least some of the bristle segments are spaced-apart to accommodate bending of the bristles.

US 8 690 907 B1 discloses a vascular treatment system and method including a plurality of self-expanding bulbs and a hypotube including interspersed patterns of longitudinally spaced rows of kerfs. Joints between woven structures and hypotubes include solder. Woven structures include patterns of radiopaque filaments measurable under x-ray. Structures are heat treated to include at least shapes at different temperatures. A catheter includes a hypotube including interspersed patterns of longitudinally spaced rows of kerfs. Heat treating systems include a detachable flange. Laser cutting systems include a fluid flow system.

### Brief Description of the Drawings

To enable better understanding of the present disclosure, and to show how the same may be carried into effect, reference will now be made, by way of example only, to the accompanying drawings, in which:
FIG. 1 shows a side view of an embolization device with first and second reinforcing sleeves disposed over the stem of the embolization device;
FIG. 2 shows a side view of an embolization device with first and second axially compressed reinforcing sleeves disposed over the stem of the embolization device;
FIG. 3 shows a side view of an embolization device with first and second straightened reinforcing sleeves disposed over the stem of the embolization device.

### Detailed Description

In one aspect of the present invention, there is disclosed an embolization device for promoting clot formation in a bodily lumen and having a contracted delivery configuration and an expanded deployed configuration. The embolization device has a stem and a plurality of flexible bristles extending radially outwardly from the stem. The stem comprises a first wire element, a second wire element, and a tubular interconnect having a lumen and connecting the first wire element to the second wire element. The first wire element and the second wire element are disposed within the lumen of the tubular interconnect. The stem further comprises a reinforcing sleeve being fixedly disposed over at least part of the tubular interconnect and fixedly disposed over at least part of one or both of the first wire element and the second wire element. The reinforcing sleeve has a lower stiffness than the tubular interconnect.

In some embodiments this may result in an embolization device where the stress concentrations during bending are more evenly distributed and the embolization device may therefore be less prone to fracture.

Throughout this disclosure the term 'embolization device' may refer to a device which may be permanently or semi-permanently implanted in a bodily lumen. Accordingly, the 'embolisation device' may be configured to be disposed within the bodily lumen for a period of time, such as a number of days, or disposed in the lumen indefinitely. To this end, the 'embolisation device' may be configured to be selectively detached from a delivery element so that it may be implanted in the bodily lumen in isolation.

Throughout this disclosure the term 'bodily lumen' may refer to the inside space within a tubular structure of the human or animal body. The 'bodily lumen' may be, for example, an artery or vein.

Throughout this disclosure the term 'contracted delivery configuration' of an element may refer to a configuration of the element which has a smaller radial extent than an `expanded deployed configuration of the element.

Throughout this disclosure the term 'stiffness' of an element may refer to the extent to which the element resists deformation in response to an applied force. The stiffness may be an extensive property of the element and dependent upon the material and its shape and boundary conditions. The stiffness k of an element may be defined as k = F/d, where F is the force on the element and d is the displacement produced by the force along the same degree of freedom.

The reinforcing sleeve may be disposed over the entire length of the tubular interconnect.

The reinforcing sleeve may be fixed to the tubular interconnect and at least part of one or both of the first wire element and the second wire element by an interference fit.

In some embodiments this may result in a more even distribution of bending stresses resulting in an embolization device less prone to fracture.

The tubular interconnect may be made from a cobalt-chromium alloy.

In some embodiments this may result in a tubular interconnect with a high specific strength.

The reinforcing sleeve may be a polymer shrink tube, which has been shrunk in a radial direction to contact the tubular interconnect and the one or both of the first wire element and the second wire element.

In some embodiments this may result in easier assembly of the embolization device. In some embodiments, this may further result in a reinforcing sleeve with a tight interference fit.

The reinforcing sleeve may be radiopaque. The reinforcing sleeve may also be doped with a radiopaque material.

Throughout this disclosure, the term 'radiopaque' may refer to the property of a substance being opaque to X-Rays or similar radiation. Any radiopaque material/object referred to herein is radiopaque to such an extent that a practitioner may easily identify the material/object during fluoroscopy. In certain embodiment, any of the radiopaque materials/objects referred to herein have a radiodensity of at least 2000 Hounsfield Units (HU), optionally at least 5000 Hounsfield Units (HU), further optionally at least 10 000 Hounsfield Units (HU), even further optionally at least 20 000 Hounsfield Units (HU).

In some embodiments this may allow a physician to see the reinforcing sleeve under fluoroscopy, to aid the physician in placement of the embolization device inside a bodily lumen. In some embodiments this may alleviate the need for a separate radiopaque marker.

The tubular interconnect may comprise a radiopaque marker.

In some embodiments this may allow a physician to see the tubular interconnect under fluoroscopy, to aid the physician in placement of the embolization device inside a bodily lumen.

The tubular interconnect may be made from a radiopaque material.

The embolization device may further comprise a radiopaque marker element disposed proximate to the tubular interconnect.

The radiopaque marker element may be abutting the tubular interconnect.

The radiopaque marker element may be disposed about the first wire element and/or the second wire element.

The plurality of flexible bristles may be grouped into a first segment of flexible bristles and a second segment of flexible bristles.

In some embodiments this may result in easier handling of the embolization device. In some embodiments, this may further allow a modular design of the embolization device.

The first segment of bristles may extend radially outwardly from the first wire element. The second segment of bristles may extend radially outwardly from the second wire element.

The radiopaque marker element may be disposed about the second wire element abutting the tubular interconnect on one end and the second segment of flexible bristles on the other end.

In some embodiments this may allow a physician to identify the position of the tubular interconnect and the second segment of flexible bristles under fluoroscopy to aid in placement of the embolization device inside a bodily lumen.

The tubular interconnect may be crimped to the first wire element and/or the second wire element.

In some embodiments this may allow the first wire element to be securely connected to the second wire element.

The tubular interconnect may be attached to the first and/or the second wire element by an adhesive.

The reinforcing sleeve may be disposed over at least part of the tubular interconnect and at least part of the first wire element. The embolization device may further comprise another reinforcing sleeve disposed over at least part of the tubular interconnect and disposed over at least part of the second wire element.

In some embodiments this may result in easier assembly of the embolization device. In some embodiments, this may further result in an embolization device where the bending stress distribution at both ends of the tubular interconnect is more evenly distributed.

The another reinforcing sleeve may be identical to the reinforcing sleeve.

The another reinforcing sleeve may be disposed over at least part of the second wire element through an intermediate radiopaque marker element.

In another aspect of the present invention, there is provided a method of assembling an embolization device for promoting clot formation in a bodily lumen and having a contracted delivery configuration and an expanded deployed configuration. The embolization device has a stem and a plurality of flexible bristles extending radially outwardly from the stem. The stem comprises a first wire element and a second wire element. The method comprises connecting the first wire element and the second wire element using a tubular interconnect having a lumen, by disposing the first wire element and the second wire element inside the lumen of the tubular interconnect. The method further comprises fixedly disposing a reinforcing sleeve over at least part of the tubular interconnect and at least part of one or both of the first wire element and the second wire element. The reinforcing sleeve has a lower stiffness than the tubular interconnect.

In some embodiments this may allow for a method of assembling an embolization device where the bending stresses are distributed more evenly which may result in an embolization device less prone to fracture.

The plurality of flexible bristles may be grouped into a first segment of flexible bristles and a second segment of flexible bristles.

In some embodiments this may result in a modular design for the embolization device. In some embodiments this may further result in easier handling and disposal of the embolization device.

The first segment of bristles may extend radially outwardly from the first wire element and the second segment of bristles may extend radially outwardly from the second wire element.

The method may further comprise, prior to connecting the first wire element to the second wire element, disposing a radiopaque marker element about the first wire element and/or the second wire element.

In some embodiments this may result in the embolization device being visible under fluoroscopy. In some embodiments this may aid a physician in placing the embolization device in a bodily lumen.

After connecting the first and second wire element, the radiopaque marker element may be proximate to the tubular interconnect. The radiopaque marker element may be abutting the tubular interconnect.

The radiopaque marker element may be disposed about the second wire element abutting the tubular interconnect on one end and the second segment of flexible bristles on the other end.

In some embodiments this may result in a physician being able to identify the position of the tubular interconnect and the segments of flexible bristles under fluoroscopy.

The step of connecting a first wire element and a second wire element may further comprise crimping the tubular interconnect to the first wire element and/or the second wire element.

In some embodiments this may result in a secure connection between the first and second wire elements.

The step of connecting a first wire element and a second wire element may further comprise attaching the tubular interconnect to the first wire element and/or the second wire element using an adhesive.

The tubular interconnect may be made from a cobalt-chromium alloy.

The tubular interconnect may be made from a radiopaque material.

The tubular sleeve may be radiopaque. The reinforcing sleeve has been doped with a radiopaque material.

In some embodiments this may allow the reinforcing sleeve to be seen under fluoroscopy, aiding in placement of the embolization device in a bodily lumen. In some embodiments this may alleviate the need for a separate radiopaque marker.

The method may further comprise axially compressing the reinforcing sleeve prior to connecting the first wire element and the second wire element.

In some embodiments this may allow the tubular interconnect to be crimped to the first wire element and the second wire element.

The method may further comprise straightening the reinforcing sleeve after connecting a first wire element and a second wire element.

In some embodiments this may allow the reinforcing sleeve to be disposed over the tubular interconnect.

After straightening the reinforcing sleeve, the reinforcing sleeve may be disposed over the entire length of the tubular interconnect.

The reinforcing sleeve may be a polymer shrink tube. Fixedly disposing the reinforcing sleeve may further comprise heating the reinforcing sleeve to shrink the reinforcing sleeve in a radial direction.

In some embodiments this may result in easier assembly of the embolization device. In some embodiments this may further result in a tight interference fit for the reinforcing sleeve.

After heating the reinforcing sleeve, the diameter of the reinforcing sleeve may be smaller than before heating.

After fixedly disposing the reinforcing sleeve, the reinforcing sleeve may contact the tubular interconnect and at least part of one or both of the first wire element and the second wire element, such that an interference fit is formed.

In some embodiments this may result in better and more even distribution of bending stresses along the stem of the embolization device.

The reinforcing sleeve may be fixedly disposed over at least part of the tubular interconnect and at least part of the first wire element and the method may further comprise fixedly disposing another reinforcing sleeve over at least part of the tubular interconnect and at least part of the second wire element.

In some embodiments this may result in easier assembly of the embolization device. In some embodiments, this may further result in an embolization device where the bending stress distributions at both ends of the tubular interconnect are more evenly distributed.

The method may further comprise axially compressing the reinforcing sleeve and the another reinforcing sleeve prior to connecting the first wire element and the second wire element.

The another reinforcing sleeve may be identical to the reinforcing sleeve.

The method may further comprise straightening the reinforcing sleeve and the another reinforcing sleeve after connecting a first wire element and a second wire element.

After straightening the reinforcing sleeve and the another reinforcing sleeve, the reinforcing sleeve may be disposed over part of the tubular interconnect and part of the first wire element. The another reinforcing sleeve may be disposed over part of the tubular interconnect and part of the second wire element.

The reinforcing sleeve and the another reinforcing sleeve may be polymer shrink tubes. Fixedly disposing the reinforcing sleeve and the another reinforcing sleeve may further comprise heating the reinforcing sleeve and the another reinforcing sleeve to shrink the reinforcing sleeve and the another reinforcing sleeve in a radial direction.

After heating the reinforcing sleeve and the another reinforcing sleeve, the diameter of the reinforcing sleeve and the another reinforcing sleeve may be smaller than before heating.

After fixedly disposing the reinforcing sleeve and the another reinforcing sleeve, the reinforcing sleeve may contact at least part of the tubular interconnect and at least part of the first wire element. The another reinforcing sleeve may contact at least part of the tubular interconnect and at least part of the second wire element, such that an interference fit is formed.

After fixedly disposing the reinforcing sleeve and the another reinforcing sleeve, the reinforcing sleeve may contact at least part of the tubular interconnect and at least part of the first wire element. The another reinforcing sleeve may contact at least part of the tubular interconnect, at least part of the radiopaque marker element and at least part of the second wire element, such that an interference fit is formed.

In a third aspect of the present disclosure there may be provided a kit comprising a first wire element having a first segment of a plurality of flexible bristles extending radially outwardly, a second wire element having a second segment of a plurality of bristles extending radially outwardly, a tubular interconnect and a reinforcing sleeve.

The kit may further comprise a radiopaque marker element.

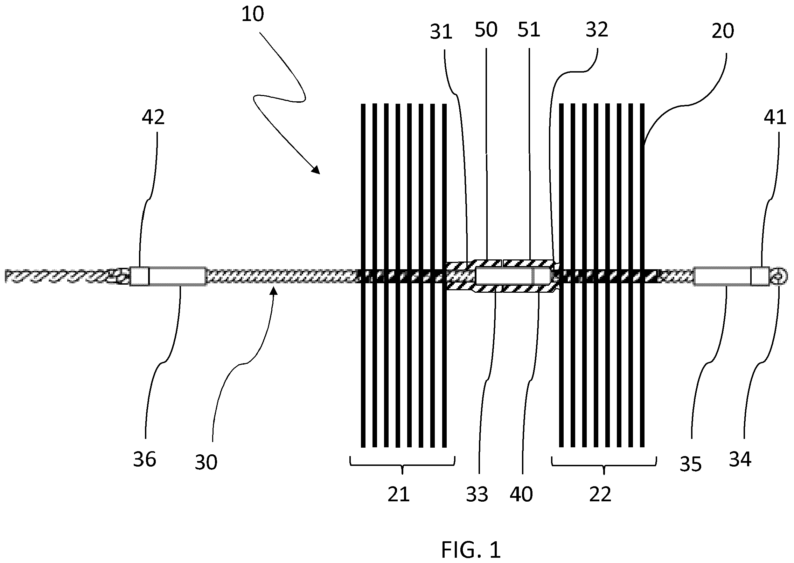

FIG. 1 shows a side view of an embolisation device 10 for promoting blood clot formation in a bodily lumen according to the present disclosure. The embolization device 10 comprises a stem 30 and a plurality of flexible bristles 20 extending radially outwardly from the stem. The embolization device 10 has an expanded deployed configuration (not shown) where the flexible bristles 20 are biased against the inner wall of a bodily lumen to anchor the embolization device 10 in the bodily lumen and a contracted delivery configuration (not shown) where the radial extent of the flexible bristles 20 is reduced compared to the expanded deployed configuration so that the embolization device 10 can be disposed inside a catheter for delivery into, or retrieval from, the bodily lumen. When the embolization device 10 is disposed in the bodily lumen in the expanded deployed configuration, the flexible bristles 20 restrict the flow of blood through the bodily lumen causing an embolus of blood to form and grow to eventually occlude the bodily lumen. FIG. 1 shows the embolization device 10 in a relaxed state where the flexible bristles 20 are straight.

The stem 30 comprises a first wire element 31 and a second wire element 32. The flexible bristles 20 are grouped into a first segment 21 which extends radially outwardly from the first wire element 31 and a second segment 22 extending radially outwardly from the second wire element 32. The first wire element 31 and the second wire element 32 are twisted wires and can be made from Nitinol or cobalt-chromium, for example.

The first wire element 31 is connected to the second wire element 32 by a tubular interconnect 33. The tubular interconnect 33 is a solid hypotube comprising a lumen which can be made from a similar material to the first and second wire elements, such as a cobalt-chromium alloy, for example. A distal end of the first wire element 31 and a proximal end of the second wire element 32 are disposed inside the lumen of the tubular interconnect 33 and the tubular interconnect 33 is crimped so as to connect the first wire element 31 to the second wire element 32. Alternatively, a biocompatible adhesive may be used to connect the tubular interconnect 33 to the first and second wire elements 31, 32.

The embolization device 10 further comprises a second tubular interconnect 35 and a third tubular interconnect 36. The second tubular interconnect 35 connects the second wire element 32 to an atraumatic distal end 34. The atraumatic distal end 34 is designed so as to not perforate or cause damage to the internal wall of a bodily lumen. The third tubular interconnect 36 connects the proximal end of the first wire element 33 to a further wire element.

The embolization device 10 also comprises a radiopaque marker element 40 disposed around the second wire element 32 and abutting the tubular interconnect 33 on one end. The radiopaque marker may be made from any material which is radiopaque to the extent that it can be easily identified by a practitioner under fluoroscopy, such as a platinum iridium compound, for example.

Intravascular procedures such as the placement of embolization device 10 at a position within a bodily lumen of a patient are usually carried out under fluoroscopy which allows a physician to see the devices inside the bodily lumen. The radiopaque marker element 40 allows a physician to visually identify the position of the tubular interconnect, the first segment of bristles 21 and the second segment of bristles 22 under fluoroscopy. This will aid the physician in placing the embolization device 10 at the desired position in the bodily lumen of a patient.

Embolization device 10 further comprises a second radiopaque marker element 41 and a third radiopaque marker element 42.

The second radiopaque marker element 41 allows the physician to visualise the distal end of the embolization device and the third radiopaque marker element 42 allows the physician to visualise of the position of the proximal end of the first wire element 31.

The embolization device 10 further comprises a first reinforcing sleeve 50 and a second reinforcing sleeve 51. The first reinforcing sleeve 50 is fixedly disposed over part of the tubular interconnect 33 and part of the first wire element 31. The first reinforcing sleeve 50 is in contact with the tubular interconnect 33 and the first wire element 31 such that an interference fit is formed. Similarly, the second reinforcing sleeve 51 is disposed over part of the tubular interconnect 33, the radiopaque marker element 40 and part of the second wire element 32. The second reinforcing sleeve 51 is in contact with the tubular interconnect 33, the radiopaque marker element 40 and the second wire element 32 such that an interference fit is formed.

The first reinforcing sleeve 50 is contacting the first segment 21 of flexible bristles 20 on one longitudinal end and the second reinforcing sleeve 51 on the other longitudinal end. The second reinforcing sleeve 51 is contacting the first reinforcing sleeve 50 at one longitudinal end and the second segment 22 of flexible bristles 20 on the other longitudinal end.

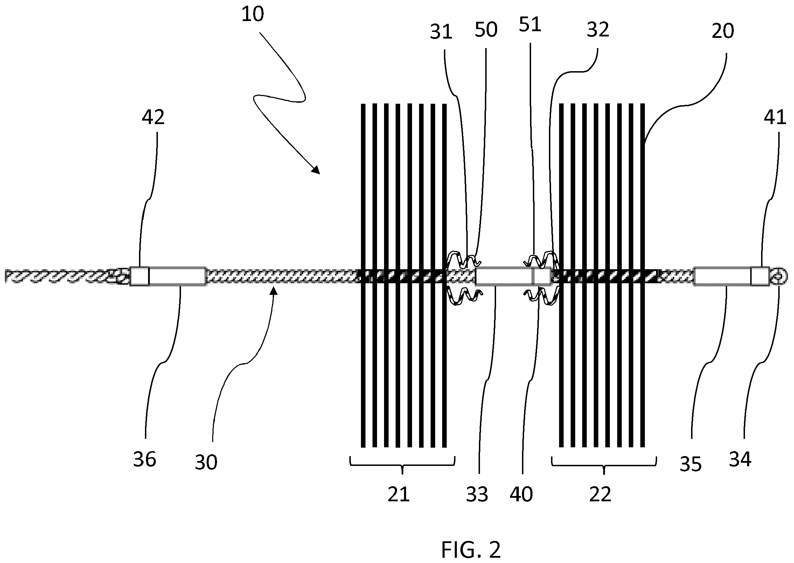

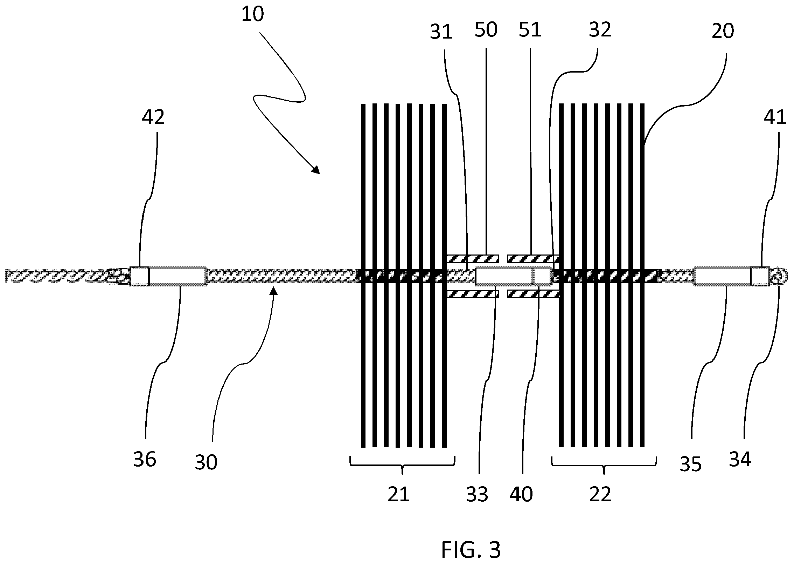

The first reinforcing sleeve 50 and the second reinforcing sleeve 51 are polymer shrink tubes which have been heated to reduce their diameter and produce an interference fit. The reinforcing sleeves may be made from a number of materials such as FEP, PTFE, Mylar or Polyolefin, for example. A method of how the reinforcing sleeves are disposed and the embolization device is assembled will be explained in more detail below with respect to FIG. 2 and FIG. 3.

The first and second reinforcing sleeves 50, 51 are disposed around the tubular interconnect 33 and at least part of the first wire element 31 and the second wire element 32. This allows the bending stresses in the first and second wire elements 31, 32 to be distributed more evenly, resulting in an embolization device which is less prone to fracture.

When placing the embolization 10 at the desired position inside the bodily lumen, the embolization device 10 disposed inside a catheter and is introduced into the body of a patient through an access site of the body. The embolization device 10 is then advanced into a distal direction and may need to undergo significant bending to navigate the tortuous blood vessels of the body to be placed at the desired position within the bodily lumen.

As noted above, the tubular interconnect 33 may be made from a similar material to the first and second wire elements 31, 32. However, due to the larger diameter of the tubular interconnect 33 and its wall thickness, the tubular interconnect 33 has a higher stiffness than the first and second wire elements.

When a bending moment is induced in the stem 30, stress concentrations occur just outside the ends of the tubular interconnect 33, due to the step change in stiffness from the tubular interconnect 33 to the wire elements. The first and second reinforcing sleeves 50, 51 have a lower stiffness than the tubular interconnect 33 and cover the tubular interconnect 33 and part of the first and second wire elements 31, 32. The ratio of stiffnesses between the tubular interconnect 33 and the first and second wire elements 31, 32 is reduced and the bending stresses are distributed more evenly along the first and second wire elements 31, 32. This will result in the stem 30 being less prone to fracture due to bending. The fatigue life of the embolization device 10 will be improved and even if fracture does occur, the first and second reinforcing sleeves 50, 51 will help to prevent the first and second wire elements 31, 32 moving relative to each other.

FIG. 2 and FIG. 3 illustrate a method of assembling the embolization device 10 of FIG. 1.

FIG. 2 shows the embolization device 10 prior to fixedly disposing the first reinforcing sleeve 50 and the second reinforcing sleeve 51.

When assembling the embolization 10, the length of the embolization device 10 can be chosen according to the specific requirements of the patient. The required number of wire elements can then be connected to form the required length of the embolization device 10.

In order to connect two wire elements such as the first wire element 31 and the second wire element 32, the first reinforcing sleeve 50 is disposed over the first wire element 31 and axially compressed. The second reinforcing sleeve 51 is then disposed over the second wire element 32 and also axially compressed. FIG. 2 shows the axially compressed reinforcing sleeves 50, 51. A radiopaque marker element 40 may be disposed about the second wire element 32. The distal end of the first wire element 31 and the proximal end of the second wire element 32 are then placed into opposite ends of the lumen of the tubular interconnect 33.

Because the first and second reinforcing sleeves 50, 51 are axially compressed, the tubular interconnect 33 is exposed. This allows a crimping tool to access the outer surface of the tubular interconnect 33 to crimp the tubular interconnect 33 to the first wire element 31 and the second wire element 32 and securely connect the first wire element 31 to the second wire element 32.

FIG. 3 shows how after the tubular interconnect 33 is crimped to the first and second wire elements 31, 32, the first and second reinforcing sleeves 50, 51 are straightened. The first reinforcing sleeve 50 is now disposed over part of the first wire element 31 and part of the tubular interconnect 33, whereas the second interconnect is now disposed over part of the second wire element 32, the radiopaque marker element 40 and part of the tubular interconnect 33. At this stage the inner diameter of the reinforcing sleeves 50, 51 is larger than the outer diameter of the tubular interconnect 33 and the first and second wire elements 31, 32.

In order to produce a tight interference fit between the reinforcing sleeves 50, 51 and the tubular interconnect 33 and the first and second wire elements 31, 32 the reinforcing sleeves 50, 51 are heated. As the reinforcing sleeves 50, 51 are polymer shrink tubes, by heating the reinforcing sleeves 50, 51 above their crystalline melting point the radial diameter of the reinforcing sleeves 50, 51 is reduced. This may be done, for example, by placing the embolization device 10 in an oven at the required temperature. In order to produce an interference fit, the inner diameter of the reinforcing sleeves 50, 51 after heating must be smaller than the outer diameter of the tubular interconnect 33 and the first wire element 31 and the second wire element 32.

After the reinforcing sleeves 50, 51 are radially shrunk in diameter and produce an interference fit with the rest of the stem 30, the embolization device 10 shown in FIG. 1 is produced.

Various modifications will be apparent to those skilled in the art. For example, the embolization device 10 may not comprise a radiopaque marker element 40.

The tubular interconnect 33 may comprise a radiopaque marker element or the tubular interconnect 33 may itself be made of a radiopaque material.

The first and second reinforcing sleeves 50, 51 may be doped with a radiopaque material to make the reinforcing sleeves 50, 51 radiopaque. This could allow removal of the radiopaque marker element 40 and allow the tubular interconnect 33 to be made from a non-radiopaque material.

The radiopaque marker element 40 may be disposed proximate to but not abutting the tubular interconnect 33.

The radiopaque marker element 40 may be disposed abutting the second segment 22 of flexible bristles 20. In that case, the second reinforcing sleeve 51 will be disposed about tubular interconnect 33 and the radiopaque marker element 40 such that it is in contact with the tubular interconnect 33 and the radiopaque marker element 40.

The first wire element 31 may also have a radiopaque marker disposed about it. This radiopaque marker may be identical to the radiopaque marker element 40.

The flexible bristles 20 may not be grouped into segments but may be disposed uniformly along the length of the stem 30.

The embolization device may be formed of any required number of wire elements connected together. Each wire element may comprise any number of segments of flexible bristles 20.

The embolization device may not have an atraumatic distal end 34.

The embolization device 10 may not have second and third radiopaque markers 41, 42. The embolization device 10 also may not have second and third tubular interconnects 35, 36.

The embolization device may only have one reinforcing sleeve. This reinforcing sleeve may be disposed over part of or the entire length of the tubular interconnect 33. This reinforcing sleeve may be disposed over part of the first wire element 31 and/or the second wire element 32.

The first and second reinforcing sleeves 31, 32 may not be polymer shrink tubes. They may be elastic rubber sleeves, for example. Alternatively, they may be polymer sleeves which are deposited onto the stem 30 of the embolization device 10 as a coating.

One of the reinforcing sleeves may be shorter than the other reinforcing sleeve. The first reinforcing sleeve 50 and the second reinforcing sleeve 51 may not be in contact, leaving part of the tubular interconnect 33 exposed.

The first reinforcing sleeve 31 may not be contacting the first segment 21 of flexible bristles 20 at one longitudinal end.

The second reinforcing sleeve 32 may not be contacting second segment 22 of flexible bristles 20 at one longitudinal end.

The first wire element 31 and the second wire element 32 may not be made from a twisted wire stem. They may be made from a solid wire stem, for example.

All of the above are fully within the scope of the present disclosure, and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

## Claims

1. An embolization device (10) for promoting clot formation in a bodily lumen and having a contracted delivery configuration and an expanded deployed configuration, the embolization device (10) having a stem (30) and a plurality of flexible bristles (20) extending radially outwardly from the stem, the stem (30) comprising:
a first wire element (31);
a second wire element (32);
a tubular interconnect (33) having a lumen and connecting the first wire element (31) to the second wire element (32), the first wire element (31) and the second wire element (32) being disposed within the lumen of the tubular interconnect (33); and
a reinforcing sleeve (50, 51) being fixedly disposed over at least part of the tubular interconnect (33) and fixedly disposed over at least part of one or both of the first wire element (31) and the second wire element (32),
**characterized in that** the reinforcing sleeve (50, 51) has a lower stiffness than the tubular interconnect (33).

2. The embolization device (10) of Claim 1, wherein the reinforcing sleeve (50, 51) is disposed over the entire length of the tubular interconnect (33).

3. The embolization device (10) of Claim 1 or 2, wherein the reinforcing sleeve (50, 51) is fixed to the tubular interconnect (33) and at least part of one or both of the first wire element (31) and the second wire element (32) by an interference fit.

4. The embolization device (10) of any preceding claim, wherein the reinforcing sleeve (50, 51) is a polymer shrink tube, which has been shrunk in a radial direction to contact the tubular interconnect (33) and the one or both of the first wire element (31) and the second wire element (32).

5. The embolization device (10) of any preceding claim, wherein the reinforcing sleeve (50, 51) and/or the tubular interconnect (33) is radiopaque and/or the embolization device (10) further comprises a radiopaque marker element (42) disposed proximate to the tubular interconnect (33).

6. The embolization device (10) of any preceding claim, wherein the plurality of flexible bristles (20) are grouped into a first segment of flexible bristles (21) and a second segment of flexible bristles (22).

7. The embolization device (10) of Claim 6, wherein the first segment of bristles (21) extends radially outwardly from the first wire element (31) and the second segment of bristles (22) extends radially outwardly from the second wire element (32) .

8. The embolization device (10) of any preceding claim, wherein the tubular interconnect (33) is crimped or attached to the first wire element (31) and/or the second wire element (32) .

9. The embolization device (10) of any preceding claim, wherein the reinforcing sleeve (50) is disposed over at least part of the tubular interconnect (33) and at least part of the first wire element (31), and wherein the embolization device (10) further comprises another reinforcing sleeve (51) disposed over at least part of the tubular interconnect (33) and disposed over at least part of the second wire element (32).

10. A method of assembling an embolization device (10) for promoting clot formation in a bodily lumen and having a contracted delivery configuration and an expanded deployed configuration, the embolization device (10) having a stem (30) and a plurality of flexible bristles (20) extending radially outwardly from the stem (30), the stem (30) comprising a first wire element (31) and a second wire element (32), the method comprising:
connecting the first wire element (31) and the second wire element (32) using a tubular interconnect (33) having a lumen, by disposing the first wire element (31) and the second wire element (32) inside the lumen of the tubular interconnect (33);
fixedly disposing a reinforcing sleeve (50, 51) over at least part of the tubular interconnect (33) and at least part of one or both of the first wire element (31) and the second wire element (32), wherein the reinforcing sleeve (50, 51) has a lower stiffness than the tubular interconnect (33).

11. The method of Claim 10, wherein the plurality of flexible bristles (20) are grouped into a first segment of flexible bristles (21) and a second segment of flexible bristles (22).

12. The method of Claim 11, wherein the first segment of bristles (21) extends radially outwardly from the first wire element (31) and the second segment of bristles (22) extends radially outwardly from the second wire element (32).

13. The method of Claims 10, 11 or 12, further comprising, prior to connecting the first and second wire element (31, 32), disposing a radiopaque marker element (40) about the first and/or second wire element (32); and/or wherein the tubular interconnect (33) is made from a radiopaque material; and/or wherein the reinforcing sleeve (50, 51) is radiopaque.

14. The method of any of Claims 10 to 13, wherein connecting a first wire element (31) and a second wire element (32) further comprises crimping or attaching the tubular interconnect (33) to the first wire element (31) and/or the second wire element (32).

15. The method of any of Claims 10 to 14, further comprising axially compressing the reinforcing sleeve (50, 51) prior to connecting the first wire element (31) and the second wire element (32).

16. The method of any of Claims 10 to 15, further comprising straightening the reinforcing sleeve (50, 51) after connecting a first wire element (31) and a second wire element (32).

17. The method of any of Claims 10 to 16, wherein the reinforcing sleeve (50, 51) is a polymer shrink tube and fixedly disposing the reinforcing sleeve (50, 51) further comprises heating the reinforcing sleeve (50, 51) to shrink the reinforcing sleeve (50, 51) in a radial direction.

18. The method of any Claim 10 to 17, wherein after fixedly disposing the reinforcing sleeve (50, 51), the reinforcing sleeve (50, 51) contacts the tubular interconnect (33) and at least part of one or both of the first wire element (31) and the second wire element (32), such that an interference fit is formed.

19. The method of any Claims 10 to 14, wherein the reinforcing sleeve (50) is fixedly disposed over at least part of the tubular interconnect (33) and at least part of the first wire element (31) and the method further comprises fixedly disposing another reinforcing sleeve (51) over at least part of the tubular interconnect (33) and at least part of the second wire element (32).

20. The method of Claim 19, wherein the reinforcing sleeve (50) and the another reinforcing sleeve (51) are polymer shrink tubes and fixedly disposing the reinforcing sleeve (50) and the another reinforcing sleeve (51) further comprises heating the reinforcing sleeve (50) and the another reinforcing sleeve (51) to shrink the reinforcing sleeve (50) and the another reinforcing sleeve (51) in a radial direction.

## Patentansprüche

1. Embolisationsvorrichtung (10) zur Förderung der Gerinnselbildung in einem Körperlumen, die eine kontrahierte Platzierungskonfiguration und eine expandierte Einsatzkonfiguration aufweist, wobei die Embolisationsvorrichtung (10) einen Schaft (30) und eine Vielzahl flexibler Borsten (20) aufweist, die sich radial nach außen vom Schaft erstrecken, wobei der Schaft (30) Folgendes umfasst:
ein erstes Drahtelement (31);
ein zweites Drahtelement (32);
eine röhrenförmige Verbindung (33), die ein Lumen aufweist und das erste Drahtelement (31) mit dem zweiten Drahtelement (32) verbindet, wobei das erste Drahtelement (31) und das zweite Drahtelement (32) innerhalb des Lumens der röhrenförmigen Verbindung (33) angeordnet sind; und
eine Verstärkungshülse (50, 51) ist, die fest über mindestens einem Teil der röhrenförmigen Verbindung (33) angeordnet ist und fest über mindestens einem Teil von einem oder beiden des ersten Drahtelements (31) und des zweiten Drahtelements (32) angeordnet ist,
**dadurch gekennzeichnet, dass**
die Verstärkungshülse (50, 51) eine geringere Steifigkeit aufweist als die röhrenförmige Verbindung (33).

2. Embolisationsvorrichtung (10) nach Anspruch 1, wobei die Verstärkungshülse (50, 51) über die gesamte Länge der röhrenförmigen Verbindung (33) angeordnet ist.

3. Embolisationsvorrichtung (10) nach Anspruch 1 oder 2, wobei die Verstärkungshülse (50, 51) an der röhrenförmigen Verbindung (33) und zumindest einem Teil von einem oder beiden des ersten Drahtelements (31) und des zweiten Drahtelements (32) durch eine Presspassung befestigt ist.

4. Embolisationsvorrichtung (10) nach einem vorstehenden Anspruch, wobei die Verstärkungshülse (50, 51) ein Polymer-Schrumpfschlauch ist, der in radialer Richtung geschrumpft wurde, um die röhrenförmige Verbindung (33) und das erste Drahtelement (31) und/oder das zweite Drahtelement (32) zu berühren.

5. Embolisationsvorrichtung (10) nach einem vorstehenden Anspruch, wobei die Verstärkungshülse (50, 51) und/oder die röhrenförmige Verbindung (33) röntgendicht ist und/oder die Embolisationsvorrichtung (10) weiter ein röntgendichtes Markierungselement (42) umfasst, das in der Nähe der röhrenförmigen Verbindung (33) angeordnet ist.

6. Embolisationsvorrichtung (10) nach einem vorstehenden Anspruch, wobei die Vielzahl der flexiblen Borsten (20) in ein erstes Segment flexibler Borsten (21) und ein zweites Segment flexibler Borsten (22) gruppiert sind.

7. Embolisationsvorrichtung (10) nach Anspruch 6, wobei das erste Borstensegment (21) sich von dem ersten Drahtelement (31) radial nach außen erstreckt und das zweite Borstensegment (22) sich von dem zweiten Drahtelement (32) radial nach außen erstreckt.

8. Embolisationsvorrichtung (10) nach einem vorstehenden Anspruch, wobei die röhrenförmige Verbindung (33) an das erste Drahtelement (31) und/oder das zweite Drahtelement (32) gecrimpt oder angebracht ist.

9. Embolisationsvorrichtung (10) nach einem vorstehenden Anspruch, wobei die Verstärkungshülse (50) über mindestens einem Teil der röhrenförmigen Verbindung (33) und mindestens einem Teil des ersten Drahtelements (31) angeordnet ist und wobei die Embolisationsvorrichtung (10) weiter eine weitere Verstärkungshülse (51) umfasst, die über mindestens einem Teil der röhrenförmigen Verbindung (33) angeordnet ist und über mindestens einem Teil des zweiten Drahtelements (32) angeordnet ist.

10. Verfahren zum Zusammenbauen einer Embolisationsvorrichtung (10) zur Förderung der Gerinnselbildung in einem Körperlumen, die eine kontrahierte Platzierungskonfiguration und eine expandierte Einsatzkonfiguration aufweist, wobei die Embolisationsvorrichtung (10) einen Schaft (30) und eine Vielzahl flexibler Borsten (20) aufweist, die sich von dem Schaft (30) radial nach außen erstrecken, wobei der Schaft (30) ein erstes Drahtelement (31) und ein zweites Drahtelement (32) umfasst, wobei das Verfahren Folgendes umfasst:
Verbinden des ersten Drahtelements (31) und des zweiten Drahtelements (32) unter Verwendung einer röhrenförmigen Verbindung (33), die ein Lumen aufweist, indem das erste Drahtelement (31) und das zweite Drahtelement (32) im Lumen der röhrenförmigen Verbindung (33) angeordnet werden;
festes Anordnen einer Verstärkungshülse (50, 51) über mindestens einem Teil der röhrenförmigen Verbindung (33) und mindestens einem Teil von einem oder beiden des ersten Drahtelements (31) und des zweiten Drahtelements (32), wobei die Verstärkungshülse (50, 51) eine geringere Steifigkeit aufweist als die röhrenförmige Verbindung (33).

11. Verfahren nach Anspruch 10, wobei die Vielzahl der flexiblen Borsten (20) in ein erstes Segment flexibler Borsten (21) und ein zweites Segment flexibler Borsten (22) gruppiert sind.

12. Verfahren nach Anspruch 11, wobei das erste Borstensegment (21) sich von dem ersten Drahtelement (31) radial nach außen erstreckt und das zweite Borstensegment (22) sich von dem zweiten Drahtelement (32) radial nach außen erstreckt.

13. Verfahren nach den Ansprüchen 10, 11 oder 12, weiter umfassend, vor dem Verbinden des ersten und des zweiten Drahtelements (31, 32), das Anordnen eines röntgendichten Markierungselements (40) um das erste und/oder zweite Drahtelement (32); und/oder wobei die röhrenförmige Verbindung (33) aus einem röntgendichten Material besteht; und/oder wobei die Verstärkungshülse (50, 51) röntgendicht ist.

14. Verfahren nach einem der Ansprüche 10 bis 13, wobei das Verbinden eines ersten Drahtelements (31) und eines zweiten Drahtelements (32) weiter das Crimpen oder das Anbringen der röhrenförmigen Verbindung (33) an dem ersten Drahtelement (31) und/oder dem zweiten Drahtelement (32) umfasst.

15. Verfahren nach einem der Ansprüche 10 bis 14, weiter umfassend das axiale Zusammendrücken der Verstärkungshülse (50, 51) vor dem Verbinden des ersten Drahtelements (31) und des zweiten Drahtelements (32).

16. Verfahren nach einem der Ansprüche 10 bis 15, weiter umfassend das Begradigen der Verstärkungshülse (50, 51) nach dem Verbinden eines ersten Drahtelements (31) und eines zweiten Drahtelements (32).

17. Verfahren nach einem der Ansprüche 10 bis 16, wobei die Verstärkungshülse (50, 51) ein Polymer-Schrumpfschlauch ist und das feste Anordnen der Verstärkungshülse (50, 51) weiter das Erwärmen der Verstärkungshülse (50, 51) umfasst, um die Verstärkungshülse (50, 51) in einer radialen Richtung zu schrumpfen.

18. Verfahren nach einem der Ansprüche 10 bis 17, wobei nach dem festen Anordnen der Verstärkungshülse (50, 51) die Verstärkungshülse (50, 51) die röhrenförmige Verbindung (33) und mindestens einen Teil von einem oder beiden des ersten Drahtelements (31) und des zweiten Drahtelements (32) berührt, so dass eine Presspassung gebildet wird.

19. Verfahren nach einem der Ansprüche 10 bis 14, wobei die Verstärkungshülse (50) fest über mindestens einem Teil der röhrenförmigen Verbindung (33) und mindestens einem Teil des ersten Drahtelements (31) angeordnet ist und das Verfahren weiter das feste Anordnen einer weiteren Verstärkungshülse (51) über mindestens einem Teil der röhrenförmigen Verbindung (33) und mindestens einem Teil des zweiten Drahtelements (32) umfasst.

20. Verfahren nach Anspruch 19, wobei die Verstärkungshülse (50) und die andere Verstärkungshülse (51) Polymer-Schrumpfschläuche sind und das feste Anordnen der Verstärkungshülse (50) und der anderen Verstärkungshülse (51) weiter das Erwärmen der Verstärkungshülse (50) und der anderen Verstärkungshülse (51) umfasst, um die Verstärkungshülse (50) und die andere Verstärkungshülse (51) in radialer Richtung zu schrumpfen.

## Revendications

1. Dispositif d'embolisation (10) pour favoriser la formation de caillot dans une lumière corporelle et présentant une configuration de distribution contractée et une configuration déployée étendue, le dispositif d'embolisation (10) présentant une tige (30) et une pluralité de poils souples (20) s'étendant radialement vers l'extérieur depuis la tige, la tige (30) comprenant :
un premier élément filaire (31) ;
un second élément filaire (32) ;
une interconnexion tubulaire (33) présentant une lumière et reliant le premier élément filaire (31) au second élément filaire (32), le premier élément filaire (31) et le second élément filaire (32) étant disposés à l'intérieur de la lumière de l'interconnexion tubulaire (33) ; et
un manchon de renforcement (50, 51) qui est disposé de manière fixe sur au moins une partie de l'interconnexion tubulaire (33) et disposé de manière fixe sur au moins une partie d'un ou des deux éléments parmi le premier élément filaire (31) et le second élément filaire (32),
**caractérisé en ce que**
le manchon de renforcement (50, 51) présente une rigidité inférieure à l'interconnexion tubulaire (33).

2. Dispositif d'embolisation (10) selon la revendication 1, dans lequel le manchon de renforcement (50, 51) est disposé sur toute la longueur de l'interconnexion tubulaire (33).

3. Dispositif d'embolisation (10) selon la revendication 1 ou 2, dans lequel le manchon de renforcement (50, 51) est fixé à l'interconnexion tubulaire (33) et à au moins une partie d'un ou des deux éléments parmi le premier élément filaire (31) et le second élément filaire (32) par un ajustement serré.

4. Dispositif d'embolisation (10) selon une quelconque revendication précédente, dans lequel le manchon de renforcement (50, 51) est un tube rétractable en polymère, lequel a été rétracté dans une direction radiale afin de venir au contact de l'interconnexion tubulaire (33) et du ou des deux éléments parmi le premier élément filaire (31) et le second élément filaire (32).

5. Dispositif d'embolisation (10) selon une quelconque revendication précédente, dans lequel le manchon de renforcement (50, 51) et/ou l'interconnexion tubulaire (33) est radio-opaque et/ou le dispositif d'embolisation (10) comprend en outre un élément de marqueur radio-opaque (42) disposé à proximité de l'interconnexion tubulaire (33).

6. Dispositif d'embolisation (10) selon une quelconque revendication précédente, dans lequel la pluralité de poils souples (20) sont regroupés en un premier segment de poils (21) souples et un second segment de poils (22) souples.

7. Dispositif d'embolisation (10) selon la revendication 6, dans lequel le premier segment de poils (21) s'étend radialement vers l'extérieur depuis le premier élément filaire (31) et le second segment de poils (22) s'étend radialement vers l'extérieur depuis le second élément filaire (32).

8. Dispositif d'embolisation (10) selon une quelconque revendication précédente, dans lequel l'interconnexion tubulaire (33) est sertie ou fixée sur le premier élément filaire (31) et/ou le second élément filaire (32).

9. Dispositif d'embolisation (10) selon une quelconque revendication précédente, dans lequel le manchon de renforcement (50) est disposé sur au moins une partie de l'interconnexion tubulaire (33) et au moins une partie du premier élément filaire (31), et dans lequel le dispositif d'embolisation (10) comprend en outre un autre manchon de renforcement (51) disposé sur au moins une partie de l'interconnexion tubulaire (33) et disposé sur au moins une partie du second élément filaire (32).

10. Procédé d'assemblage d'un dispositif d'embolisation (10) pour favoriser la formation de caillot dans une lumière corporelle et présentant une configuration de distribution contractée et une configuration déployée étendue, le dispositif d'embolisation (10) présentant une tige (30) et une pluralité de poils souples (20) s'étendant radialement vers l'extérieur depuis la tige (30), la tige (30) comprenant un premier élément filaire (31) et un second élément filaire (32), le procédé comprenant :
le fait de relier le premier élément filaire (31) et le second élément filaire (32) en utilisant d'une interconnexion tubulaire (33) présentant une lumière, en disposant le premier élément filaire (31) et le second élément filaire (32) à l'intérieur de la lumière de l'interconnexion tubulaire (33) ;
le fait de disposer de manière fixe un manchon de renforcement (50, 51) sur au moins une partie de l'interconnexion tubulaire (33) et au moins une partie d'un ou des deux éléments parmi le premier élément filaire (31) et le second élément filaire (32), dans lequel le manchon de renforcement (50, 51) présente une rigidité inférieure à l'interconnexion tubulaire (33).

11. Procédé selon la revendication 10, dans lequel la pluralité de poils souples (20) sont regroupés en un premier segment de poils (21) souples et un second segment de poils (22) souples.

12. Procédé selon la revendication 11, dans lequel le premier segment de poils (21) s'étend radialement vers l'extérieur depuis le premier élément filaire (31) et le second segment de poils (22) s'étend radialement vers l'extérieur depuis le second élément filaire (32).

13. Procédé selon les revendications 10, 11 ou 12, comprenant en outre, avant de relier le premier et le second élément filaire (31, 32), le fait de disposer un élément de marqueur radio-opaque (40) autour du premier et/ou second élément filaire (32) ; et/ou dans lequel l'interconnexion tubulaire (33) est réalisée à partir d'un matériau radio-opaque ; et/ou dans lequel le manchon de renforcement (50, 51) est radio-opaque.

14. Procédé selon l'une quelconque des revendications 10 à 13, dans lequel la liaison d'un premier élément filaire (31) et d'un second élément filaire (32) comprend en outre le fait de sertir ou fixer l'interconnexion tubulaire (33) sur le premier élément filaire (31) et/ou le second élément filaire (32).

15. Procédé selon l'une quelconque des revendications 10 à 14, comprenant en outre le fait de compresser axialement le manchon de renforcement (50, 51) avant de relier le premier élément filaire (31) et le second élément filaire (32).

16. Procédé selon l'une quelconque des revendications 10 à 15, comprenant en outre le fait de redresser le manchon de renforcement (50, 51) après la liaison d'un premier élément filaire (31) et d'un second élément filaire (32).

17. Procédé selon l'une quelconque des revendications 10 à 16, dans lequel le manchon de renforcement (50, 51) est un tube rétractable en polymère et la disposition de manière fixe du manchon de renforcement (50, 51) comprend en outre le fait de chauffer le manchon de renforcement (50, 51) afin de rétracter le manchon de renforcement (50, 51) dans une direction radiale.

18. Procédé selon l'une quelconque des revendications 10 à 17, dans lequel après avoir disposé de manière fixe le manchon de renforcement (50, 51), le manchon de renforcement (50, 51) vient au contact de l'interconnexion tubulaire (33) et d'au moins une partie d'un ou des deux éléments parmi le premier élément filaire (31) et le second élément filaire (32), de telle sorte qu'un ajustement serré est formé.

19. Procédé selon l'une quelconque des revendications 10 à 14, dans lequel le manchon de renforcement (50) est disposé de manière fixe sur au moins une partie de l'interconnexion tubulaire (33) et au moins une partie du premier élément filaire (31) et le procédé comprend en outre le fait de disposer de manière fixe un autre manchon de renforcement (51) sur au moins une partie de l'interconnexion tubulaire (33) et au moins une partie du second élément filaire (32).

20. Procédé selon la revendication 19, dans lequel le manchon de renforcement (50) et l'autre manchon de renforcement (51) sont des tubes rétractables en polymère et la disposition de manière fixe du manchon de renforcement (50) et de l'autre manchon de renforcement (51) comprend en outre le fait de chauffer le manchon de renforcement (50) et l'autre manchon de renforcement (51) afin de rétracter le manchon de renforcement (50) et l'autre manchon de renforcement (51) dans une direction radiale.
